(19) Europäisches Patentamt
European Patent Office
Office européen des brevets

(11) **EP 1 028 319 A2**

(12) **EUROPEAN PATENT APPLICATION**

(43) Date of publication:
**16.08.2000 Bulletin 2000/33**

(51) Int. Cl.[7]: **G01N 33/92**, B01D 39/00,
B01D 39/20

(21) Application number: **00109181.8**

(22) Date of filing: **15.07.1991**

(84) Designated Contracting States:
**AT BE CH DE DK ES FR GB GR IT LI LU NL SE**

(30) Priority: **16.07.1990 US 554147**
**12.07.1991 US 729447**

(62) Document number(s) of the earlier application(s) in accordance with Art. 76 EPC:
**91913726.5 / 0 539 495**

(71) Applicant:
**CHOLESTECH CORPORATION**
**Hayward, CA 94545-3877 (US)**

(72) Inventor: **Jones, Ronald M.**
**Mountain View, California 94040 (US)**

(74) Representative:
**Hess, Peter K., Dipl.-Phys. et al**
**Patent- und Rechtsanwälte**
**Bardehle - Pagenberg - Dost**
**Altenburg - Geissler -Isenbruck**
**Galileiplatz 1**
**81679 München (DE)**

Remarks:
This application was filed on 08 - 05 - 2000 as a divisional application to the application mentioned under INID code 62.

(54) **Device and method for measuring HDL-cholesterol emplying precipitation on a solid-phase**

(57) An assay device (14) for measuring the concentration of a soluble analyte, such as HDL-associated cholesterol, in a fluid sample containing interfering compounds, such as LDL- or VLDL-associated cholesterol, which can be selectively precipitated. The device includes a sieving matrix (26) capable of separating soluble and precipitated material migrating through the matrix, and a reservoir (34) which holds a precipitating agent which is effective, within a given concentration range, to selectively precipitate the interfering compounds. The reservoir is designed to delay the release of agent, on contact with the fluid sample, to maintain the concentration of precipitating agent in contact with the fluid sample within the given concentration range. The device additionally includes an assay pad (64, 66, 68, 70) in which the soluble analyte present in the fluid sample can be assayed. A method of separating high-density lipoproteins (HDL) from low- and very-low-density lipoproteins (LDL and VLDL) employs a coated glass fiber matrix which inhibits binding of HDL as a blood fluid sample containing HDL and precipitated LDL and VLDL passes through the matrix.

Fig. 1a

EP 1 028 319 A2

**Description**

1. Field of the Invention.

The present invention relates to a solid phase precipitation diagnostic device and to a method of separating high density lipoproteins (HDL) from low density lipoproteins (LDL) in a blood-fluid sample.

2. References

Arai, F. et al. U.S. Patent No. 4,826,761, issued May 2, 1989.
Bachorik, P.S., et al., in Methods in Enzymology, Vol. 129, Academic Press (1986), pp 78-100.
Figueras et al. U.S. Patent No. 4,144,306, issued March 13, 1979.
Goldfarb, A. et al. U.S. Patent No. 3,464,413, issued September 2, 1969.
Vogel et al. U.S. Patent No. 4,477,575, issued October 16, 1984.
Zaffaroni, A. U.S. Patent No. 3,797,494, issued March 19, 1974.
Ziegenhorn et al. U.S. Patent No. 4,544,630, issued October 1, 1985.

3. Background of the Invention

There is a trend toward widespread testing of blood and other body-fluids for analytes which are predictive of health conditions, such as risk of coronary disease. The amount of cholesterol present in the blood is one factor which is related to the risk of coronary artery disease.

Cholesterol circulates in the blood predominantly in the protein-bound form. The proteins which transport cholesterol are the lipoproteins, which are subdivided into three classes based on their density. The very-low density lipoproteins (VLDL) are triglyceride-rich lipoproteins which are synthesized in the liver and ultimately converted to low-density lipoproteins (LDL), which transport most of the plasma cholesterol in humans. The high-density lipoproteins (HDL) are lipoproteins which are involved in the catabolism of triglyceride-rich lipoproteins, and in the removal of cholesterol from peripheral tissues and transport to the liver. An inverse relationship between serum HDL levels and risk of coronary disease has been established. In particular, if the proportion of serum cholesterol associated with HDL is low, the risk of coronary disease is increased.

In view of the importance of relative serum cholesterol levels in risk assessment and management of atherogenic disease, considerable effort has been spent screening large populations of both normal and high-risk individuals for serum levels of HDL, LDL, as well as total cholesterol and triglycerides. The effectiveness of treatments of high-risk individuals has been monitored by regular testing of serum levels of cholesterol in the various lipoprotein compartments.

One method for LDL and HDL cholesterol testing is based on the selective precipitation of non-HDL lipo-proteins in serum by polyanionic compounds, such as dextran sulfate, heparin, and phosphotungstate, in the presence of a group-II cation, such as $Mg^{2+}$, $Mn^{2+}$, and $Ca^{2+}$. The specificity and degree of precipitation are dependent on a variety of factors, including the type and concentration of the polyanion/metal agent. In general, the order of precipitation of serum cholesterol particles, with increasing concentration of polyanion is VLDL, LDL, and HDL. HDL usually remains soluble at concentrations of heparin or dextran sulfate which completely precipitate lower density particles, although minor apoE species of HDL may be co-precipitated with lower density particles.

By selective precipitation of lower density particles, HDL serum cholesterol levels can be determined. The HDL value, along with total serum cholesterol and triglycerides measurements can then be used to calculate LDL according to the relationship:

$$\text{LDL cholesterol} = \text{total cholesterol} - \text{triglycerides}/5 - \text{HDL cholesterol}$$

In a typical lipid assay procedure, a small volume of blood is drawn and centrifuged to produce a clear plasma or serum fluid sample. The fluid sample is then aliquoted into several assay tubes, for determination of (a) total serum cholesterol, (b) triglycerides, and (c) HDL cholesterol. The HDL sample is precipitated, as above, and the lower density particles are removed by filtration or centrifugation prior to cholesterol detection. The samples are then reacted with an enzyme mix containing cholesterol esterase, cholesterol oxidase, peroxidase and a dye which can be oxidized to a distinctly colored product in the presence of $H_2O_2$. The tubes may be read spectrophotometrically, and the desired total, HDL and LDL cholesterol values determined.

Despite the accuracy and reliability which can be achieved with the liquid-phase cholesterol assay just described, the assay has a number of limitations for use in widespread screening. First, the method uses a venous blood sample, requiring a trained technician to draw and fractionate the blood sample, and aliquot the treated blood to individual assay tubes. At least one of the sample tubes (for HDL determination) must be treated with a precipitating agent, and further processed to remove precipitated material. Although some of these procedures can be automated, analytical machines designed for this purpose are expensive and not widely available outside of large hospitals.

The present invention provides an assay device which substantially overcomes many of the above-mentioned problems associated with liquid-assay procedures for measuring serum cholesterol levels. In one embodiment, the device is designed for measuring the concentration of HDL-associated cholesterol, in a blood sample also containing LDL and VLDL particles. The device includes a sieving matrix capable of separating soluble and precipitated lipoproteins as a fluid sample migrates through the matrix. A reservoir associated with the matrix is designed to release a precipitating agent, for selectively precipitating LDL and VLDL, as fluid sample is drawn into and through the matrix. This allows HDL separation from the precipitated lipoproteins, based on faster HDL migration through the sieving matrix. The fluid sample thus depleted of non-HDL lipoproteins is assayed for cholesterol.

4. Summary of the Invention

It is one object of the invention to provide an assay device and method which substantially overcome or reduce above-mentioned problems associated with liquid-assay procedures for assaying serum LDL, HDL, and total cholesterol values.

A general object of the invention is to provide an improved solid-phase assay device for measuring a soluble analyte in a fluid sample which also contains a soluble assay-interfering compound.

In one embodiment, the invention includes an assay device for use in measuring serum cholesterol associated specifically with HDL. A precipitating agent selectively precipitates VLDL and LDL, so that HDL-associated cholesterol can be measured without interference from these sources.

The assay device of the invention includes a sieving matrix which separates soluble from precipitated material in a fluid sample, as the sample flows through the matrix from a sample-application site to a sample-collection site in the matrix. A reagent reservoir in the device is designed to slowly release a precipitating agent into the matrix, as fluid sample flows into and through the matrix. In a preferred embodiment, the sieving matrix is composed of a matrix of glass fibers, and the precipitating agent is coated onto the fibers. The slow release of precipitating agent from the reservoir provides a concentration of precipitating agent in the matrix which is effective to precipitate interfering compound(s) in the sample, without precipitating the analyte being assayed. Also included in the device is an assay pad to which fluid sample collected at the sample-collection site on the matrix can be transferred, for determination of analyte concentration present in the fluid sample.

In a preferred embodiment, the precipitating agent includes the combination of a polyanionic compound and a group-II metal cation, and the release means is effective to release the polyanionic compound at a rate which is sufficient to provide a concentration of the compound which selectively precipitates VLDLs and HDLs. Preferred polyanionic compounds for use in the reagent reservoir include sulfated polysaccharides, heparin, and phosphotungstiC acid, and the class II metal is selected from the group consisting of $Mg^{2+}$, $Mn^{2+}$, and $Ca^{2+}$. A preferred combination includes final concentrations of 0.5-1.0 mg/ml dextran sulfate and 0.045-0.15 M $Mg^{2+}$. These preferred precipitation agent formulations are particularly useful in the assay of HDL-associated cholesterol.

In another embodiment, the device includes a coating material applied to the matrix which prevents binding of the soluble analyte to the matrix in the presence of such precipitating agent. Preferred coatings effective in inhibiting binding of HDLs to glass fibers include hydrophilic polymers, such as polyvinyl alcohol, polyvinyl pyrrolidine, polyethylene glycol, polypropylene glycol, polyethylenimine, polyvinyl sulfonic acid, poly(3-hydroxybutyric acid), polyacrylic acid, poly(3-hydroxyvaleric acid), poly(4-styrene sulfonic acid), poly(2-acrylamido sulfonic acid), or poly-L-glutamate.

Another preferred coating for a matrix composed of a network of glass fibers having surface silyl groups is one in which the coating, including the surface silyl groups, is composed of silyl ethers, particularly silyl mono- or diethers of bis(hydroxyethyl)aminopropyltriethoxy silane.

In another aspect, the invention includes a method of separating HDLs from LDLs and VLDLs in a blood fluid sample. In the method, the fluid blood sample is mixed with a quantity of precipitating agent which selectively precipitates the LDLs and VLDLs. The sample is then passed through a matrix of glass fibers, which have a coating which inhibits binding of HDL to the fibers.

Preferably, the precipitating agent used in the method of the invention includes a polyanionic compound and a group-II metal cation. Polyanionic compounds which are particularly useful in the method of the invention include sulfated polysaccharides, heparin, and phosphotungstic acid. The class II metal is preferably $Mg^{2+}$, $Mn^{2+}$, or $Ca^{2+}$.

In one embodiment of the method of the invention, the coating used to prevent binding of soluble HDL to the glass fiber matrix in the presence of precipitating agent is preferably a hydrophilic polymer selected from the group consisting of polyvinyl alcohol, polyvinyl pyrrolidine, polyethylene glycol, polypropylene glycol, polyethylenimine, polyvinyl sulfonic acid, poly(3-hydroxybutyric acid), polyacrylic acid, poly(3-hydroxyvaleric acid), poly(4-styrene sulfonic acid), poly(2-acrylamido sulfonic acid), or poly-L-glutamate.

In another embodiment, the matrix comprises glass fibers having surface silyl groups, and the coating is composed of silyl ethers. Preferred silyl etheres include silyl mono- or di-ethers of bis(hydroxyethyl)aminopropyltriethoxy silane.

5. Brief Description of the Drawings

Figures 1A and 1B are a side view of a multi-analyte assay device constructed in accordance with the invention and a crossection through the sideview, taken at the line indicated, respectively;
Figure 2 is an enlarged fragmentary view of the central strip region of the assay device shown in Figure 1;
Figures 3a, 3b, and 3c are enlarged views of three types of slow-release reservoir employed in the device;
Figure 4 illustrates release of precipitating agent and precipitation of LDL (open squares) and VLDL (open triangles) in a serum sample (left side of the figure) and the separation of HDL (solid circles) particles from precipitated LDL and VLDL which form after migration through the strip in the device (center of the figure);
Figures 5A-5C illustrate methods for coating a glass fiber surface (5A) with a silylating agent (5B) or with a hydrophilic polymer (5C);
Figure 6 is a bar graph showing measured HDL cholesterol values after HDL separation on a glass fiber matrix with various treatments and glass fiber coatings;
Figure 7 is a plan view of a strip in the assay device designed for determination of serum cholesterol associated with HDL;
Figure 8 is a plot illustrating the determination of HDL cholesterol concentrations using a two reference standard curve in accordance with use of the method and device of the invention;
Figures 9a and 9b illustrate a plan view of a strip in the assay device designed for determination of multiple analytes, one of which is HDL, and a fragmentary crossection through the strip, respectively;
Figure 10 is a plot illustrating the determination of total cholesterol, HDL, and serum triglyceride from the assay device in accordance with the method of the invention; and
Figure 11 is a plot comparing HDL cholesterol measured in accordance with the present invention and by a standard liquid-phase approach, where the straight line in the figure indicates ideal conformity between the two tests.

6. Detailed Description of the Invention

A. Assay Device

This section describes an assay device suitable for use with single or multiple analytes designed for measuring a soluble analyte in a fluid sample which also contains one or more interfering compounds. An interfering compound may be one which reacts with reagents used for detecting the analyte of interest, inhibits such reagents, or non-specifically interferes with the level of signal produced in the analyte-specific reaction. The interfering compound is also one which can be selectively precipitated (or aggregated) under conditions which do not precipitate the soluble analyte molecule or molecular complex. Selective precipitation is produced by exposure of the sample to a precipitating agent Within a given concentration range. The interfering compound may be incompletely precipitated below the concentration range; above this range, precipitation of the analyte may occur.

One exemplary analyte for detection by the present invention is high density lipoprotein-associated cholesterol (HDL cholesterol). As discussed above, HDL is one of the classes of lipoproteins which carries cholesterol in the blood. Cholesterol levels associated with HDL, and with low-density lipoproteins (LDL), provide a strong correlation with cardiovascular disease. Compounds that interfere in the HDL assay are primarily the non-HDL serum lipoproteins, including chylomicrons, VLDL and LDL. As will be discussed below, the non-HDL lipoproteins can be selectively precipitated by a variety of polyanionic compounds, in combination with group-II metal cations, within selected concentration ranges of the precipitating components.

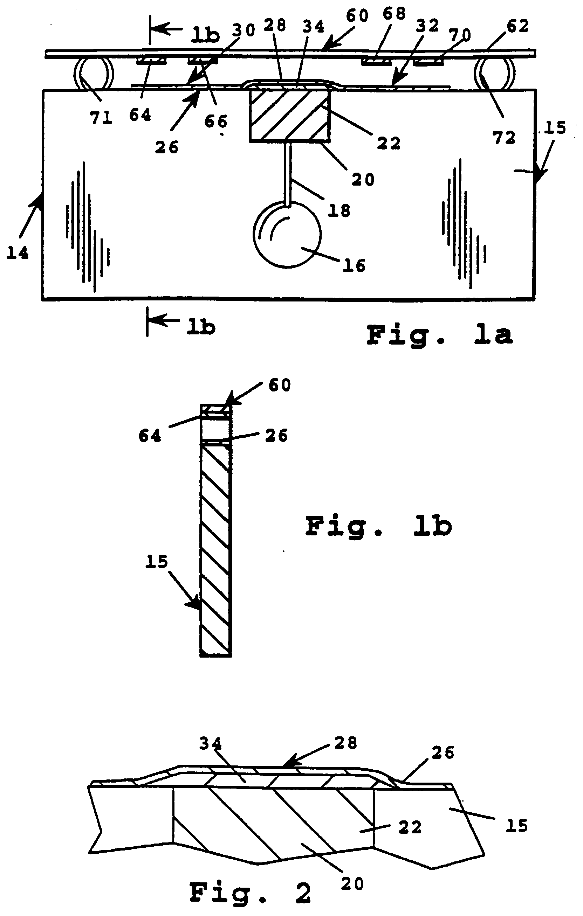

Figure 1a illustrates a multiple-analyte assay device 14 constructed in accordance with the present invention. The device is designed particularly for determining multiple analytes using a small volume of blood sample, typically between 10-50 µl of blood. The general assay configuration has been described in co-owned U.S. patent application for "Multi-Analyte Assay Device", Serial No. 503,371, filed April 2, 1990, which is herein incorporated by reference and is particularly suitable for determination of multiple blood-sample assays.

Device 14 generally includes a support 15 which defines a well 16 dimensioned and sized to receive a quantity of a blood sample, and typically between about 25-50 µl of blood. A capillary conduit 18 formed in the plate is provided at the base of the well and communicates with notched region 20 formed in the upper edge of the support. The support is preferably a thin plastic plate or the like, with the well, tube and notched region formed by standard molding or machining methods.

A sieving pad 22 carried in region 20 functions to partially remove large particulate matter (including blood cells) as the sample migrates through the matrix in a direction away from the well, bottom-to-top as illustrated in Figure 1a. Pad 22 is formed of a fibrous matrix material designed to draw aqueous fluid by surface wetting, and to retard the movement of blood cells as the blood samp le is drawn through the matrix. That is, the pad serves as a chromatographic medium for separating cell-size particles from soluble serum components on the basis of different migration rates through the medium.

A variety of fibrous materials, such as are used in fibrous-mat filters, including cellulose, cellulose acetate, and glass fibrous matrices, are suitable materials for the sieving pad. The fibers may be crosslinked, if desired, by chemical crosslinking, heat fusion, or the like. Also suitable are porous substrates, such as sintered glass, fused polymer beads, and the like whose wettability and dimension of interstices are such as to promote movement of an aqueous medium into the matrix by surface wetting. One exemplary filter is a glass fiber filter, such as a GF/D or PD008 filter supplied by Whatman, having a packing density of about 0.16 g/cm$^3$. The strip is cut to side dimensions of about 3 X 8 mm, and a thickness of about 1 mm. The pad is dimensioned to absorb a defined volume of fluid sample, typically about 3-25 µl, and preferably between about 15-25 µl.

Sieving pad 22 may additionally contain red blood cell capture reagents such as lectins, antibodies specific for red blood cell surface membrane proteins, thrombin, or ion exchange agents. Alternatively, the upper surface of pad 22 may be covered by a microporous membrane designed to filter out blood cells and other particulate matter present in the fluid sample. Where the device is used for assaying total cholesterol or other lipid components which may be associated with large lipoprotein bodies in the blood, e.g., HDL, LDL, and VLDL, the pore sizes in such a membrane are selected to filter out blood cells, but allow passage of these molecules. One preferred membrane is a polycarbonate membrane available from Nucleopore (Livermore, CA) and having a 1 micron pore size.

With reference particularly to Figure 2, which is an enlargement of the central portion of the assay device, the sieving pad, 22, in turn, is covered by an elongate strip or matrix 26 which is attached to and extends along an interior portion of the plate's upper edge. A matrix 26 serves to distribute fluid sample from a central sample-application region 28 of the matrix strip, to opposite sample-collection regions 30, 32 adjacent the ends of the matrix (Figure 1). The matrix is preferably formed of a fibrous material, such as employed in pad 22, which is capable of drawing fluid through the strip by capillary flow.

The packing density and thickness of the matrix are such as to absorb and distribute small fluid volumes, e.g., 10-25 µl, supplied to the sample-application region of the strip to the sample-collection regions of the strip. The matrix has a preferred packing density between about 0.16 g/cm$^3$ and 4.0 g/cm$^3$. One exemplary matrix material is BSB-20 glass fiber filter available from Whatman having a packing density of about 0.2 gm/cm$^3$, a width of about 3 mm, a length of about 3 cm, and a thickness of about 0.12 mm. The glass fibers in the separation matrix are prepared to contain a coating which is effective to prevent binding of HDL to the glass fibers in the presence of a precipitating agent, as will be described below.

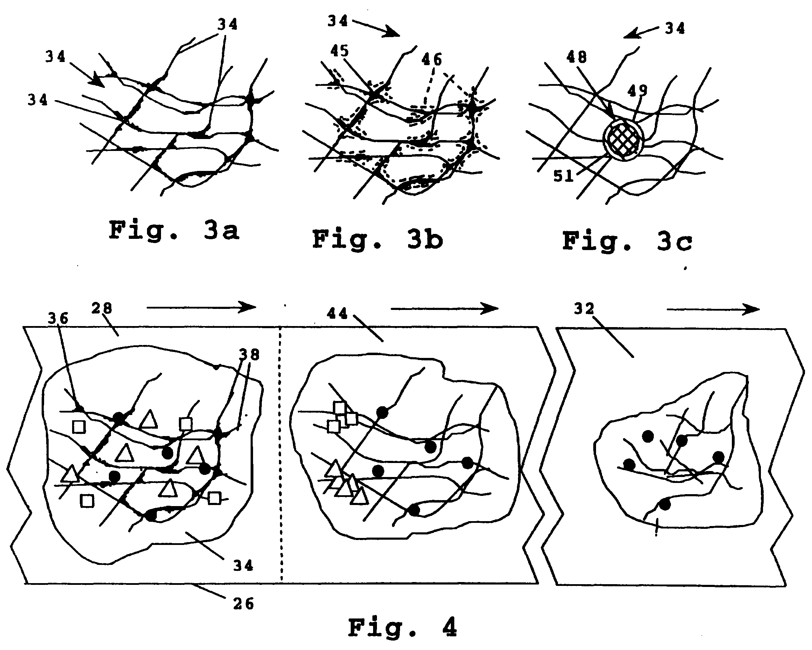

With continued reference to Figure 2, a reagent reservoir 34 in the device contains a precipitating agent used to selectively precipitate interfering compounds, such as LDL and VLDL particles in the fluid sample, as fluid sample is drawn into the sample-application region of the matrix. Reservoir 34 includes a fibrous web (illustrated in Figures 3a, 3b, and 3c) Which is interposed between sieving pad 22 and the central sample application region 28 of matrix 26. As seen, the web extends somewhat beyond the edges of region 20, ensuring that fluid sample being drawn from pad 22 into matrix 26 is also drawn through the reservoir. The web may be formed of a fibrous filter material such as employed in pad 22 or matrix 26. One exemplary material is a glass fiber filter having a packing density of about 0.2 gm/cm$^3$, a thickness of about 0.12 mm, and length and width dimensions of about 4 and 3 mm, respectively. As described in detail below, Figures 3a, 3b, and 3b show embodiments of an enlarged portion of reagent reservoir, illustrating its web construction. Although the reservoir is shown here as separate from matrix 26, it will be appreciated that the reservoir may be integrally formed with the matrix, by incorporating the precipitating agents into a central portion of the matrix.

Reagent reservoir 34 is designed to release the precipitating agent at a rate which maintains the concentration of agent in the fluid sample entering the matrix, and more specifically, at least the first 10% and preferably 20-40% of the total fluid sample which is drawn into the matrix between the sample-application and sample-collection sites on the matrix, within a concentration range which is effective to selectively precipitate or aggregate the non-HDL compounds in the fluid sample.

The precipitating agent is any chemical component or components (a) whose ability to precipitate the interfering compound is concentration dependent, and (b) which is released from the reservoir into the fluid sample in soluble form. The agent may be a salt, producing a salting out precipitation effect, an acid or a base, producing a selective pH-dependent precipitation effect, or more typically, a compound or compounds which form intermolecular aggregates with the interfering compound(s). The preferred precipitating agent, for use in selective precipitation of non-HDL serum lipoproteins is a sulfonated polysaccharide, such as dextran sulfate (which has a typical molecular weight of 50,000 daltons) in combination with magnesium acetate or chloride, buffered to maintain neutral pH.

As discussed above, lipoproteins can be selectively precipitated by a variety of precipitating agents, including combinations of selected polyanionic compounds and divalent cations. The use of these agents in lipoprotein precipitation has been reviewed (Bachorik, P.S., et al., in Methods in Enzymology, Academic Press (1986), pp 78-100). The agents which have been most widely used are (a) heparin-$Mn^{2+}$ or heparin-$Ca^{2+}$, (b) dextran sulfate-$Mg^{2+}$, (c) phosphotungstate-$Mg^{2+}$, and (d) polyethylene glycol.

The heparin agent including heparin and a divalent metal is effective at a heparin concentration of 1 mg/ml, and a concentration of up to 1 mg/ml does not precipitate HDL. $Mn^{2+}$, typically in the form of the $MnCl_2$ salt, should be at least about 0.04 M for complete precipitation of non-HDL (apoB-containing) lipoproteins, and preferably between about .006 and .092 M $Mn^{2+}$. Similar concentrations of $Ca^{2+}$ can be substituted for $Mg^{2+}$.

The dextran sulfate agent including dextran sulfate and $Mg^{2+}$ preferably employs dextran sulfate of molecular weight about 50 kd (kilodaltons). At higher molecular weights e.g., 500 kd, a significant amount of HDL is precipitated. At lower molecular weights, e.g., 15 kd, the apoB lipoproteins are incompletely precipitated. The optimal dextran sulfate concentration for selective precipitation of non-HDL particles is about 0.91 mg/ml, and concentration up to 10 mg/ml do not precipitate HDL. The concentration of $Mg^{2+}$, typically in the form of $MgCl_2$ is between about 0.045 and 0.15 M.

The phosphotungstate agent includes a water-soluble phosphotungstate and $Mg^{2+}$, typically in the form of $MgCl_2$. Concentrations of the agent effective to selectively precipitate non-HDL lipoproteins are between 2-8 mg/ml of phosphotungstate and between about 0.025 and 0.075 M Mg salt.

The precipitating agent is typically formulated in aqueous buffer, such as HEPES buffer to a desired concentration which allows infusion of the reservoir web with a selected amount of precipitating agent. The total amount of agent in the reservoir is calculated from the estimated solvent-front volume in the assay device which will contain precipitating agent, at the desired concentration of Precipitating agent, and the total amount of precipitating agent required to produce that concentration. The formulation applied to the web may include a binder, as described with reference to Figure 3a, below, or precipitating agent alone, which is coated with a slow-release coating. Details of a reservoir containing a dextran sulfate-$MgCl_2$ precipitating agent are given in Example 1.

The binder material and proportion of binder to precipitating agent in the formulation are selected to provide gradual particle dissolution over the period when fluid sample is drawn into the matrix, and more specifically, when the first 10-40% or more of total fluid sample is drawn into the matrix. Preferred polymer binders are linear polyoxides, polypropylene oxides, polyethylene imines, polyacrylic acid, polyacrylamide, polymethacrylamide, polymethacrylic acid, polyvinylacetate, polyvinylpyrrolidone, polyvinyloxazolidone, and a variety of water-soluble hydroxyl polymers, such as natural gums, gelatin, and water-soluble cellulose compounds, such as methylcellulose and hydroxymethylcellulose, and co-polymers and blends of these polymers). These polymers are generally available commercially in a variety of molecular weight sizes. Binder formulations can be prepared by blending The binder with the precipitating agent, typically in a weight ratio of between about 1:10 to 1:1 precipitating agent:binder. The dried binder formulation typically contains between about 1:10 1:1 agent:binder, according to nature of the binder and desired dissolution rate of the reservoir. It is noted, however, that the binder itself may be the precipitating agent or one of The components of the agent, such as a dextran sulfate polymer in a precipitating agent composed of dextran sulfate and $Mg^{2+}$.

Figures 3a, 3b, and 3c show enlarged views of embodiments of reservoir 34, particularly illustrating the web-like construction of the preferred embodiment. In the embodiment illustrated in Figure 3a, the precipitating agent is formulated in a binder and dried onto the fibers of the web. That is, the fibers of the web, indicated at 38, are encased in a dried irregular deposit 36 of a binder/precipitating agent mix. The binder material and proportion of binder to precipitating agent in the formulation are selected to provide gradual particle dissolution over the period when fluid sample is drawn into the matrix, and more specifically, when the first 10-40% or more of total fluid sample is drawn into the matrix. To prepare the reservoir, the web is infused with a suspension of the binder formulation containing a known quantity of the precipitating agent. The web is then dried, e.g., under vacuum, or by heating, resulting in web coating as shown in Figure 3a. Example 1 describes the preparation of a reservoir of this type containing dextran sulfate and $MgCl_2$.

In a second embodiment, illustrated in Figure 3b, the precipitating agent is infused into the web and dehydrated, forming a deposit 45 on the fibers of the web. This deposit is then coated with a water-soluble material, typically a water-soluble polymer such as mentioned above, forming a slow-release coating 46 (shown in dotted lines) on the deposit which acts to delay dissolution of the deposit on contact with fluid sample. The coating may be applied by spraying, dipping, or dehydration from a non-aqueous solvent mixture applied to the web and deposit. The coating is preferably applied so as to achieve different thicknesses of coating throughout the web, to produce a continuum of released agent as sample material flows through the reservoir.

Figure 3c illustrates a microsphere or microcapsule type of reservoir particle 48 in the reservoir. The microspheres are composed of a microporous polymeric outer shell 49 and an encapsulated interior region 51 which contains the precipitating agent. The microspheres with encapsulated agent are prepared according to known methods such as described in U.S. Patents No. 3,797,494 and 3,464,413, and embedded in the web. The porosity of the microspheres is such as to exclude the analyte compound of interest, to prevent exposure of analyte to the high concentration of the precipitating agent within the microspheres.

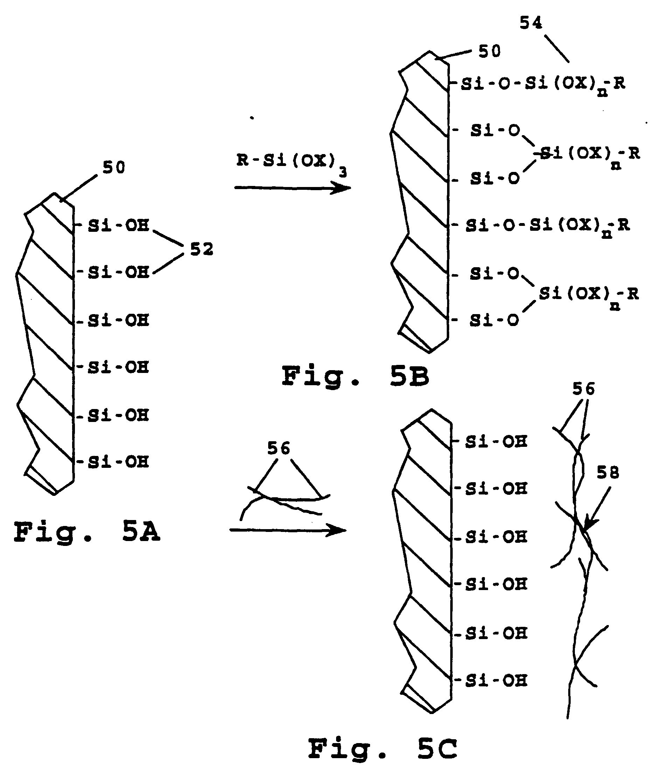

Figure 4 illustrates the operations of selective precipitation and separation which occur between sample-application and sample-collection regions in matrix 26, during an assay for determination of HDL in a serum sample. The different lipoproteins in the sample are indicated by solid circles (HDL), open squares (VLDL), open triangles (LDL), and clusters of the units (aggregates). The fluid sample flows through the matrix from left to right, as illustrated, indicated by the arrows.

The left portion of Figure 4 shows the sample-application region 28 of the matrix, with the cutaway portion showing the fibers 38 of the reagent reservoir 34 in the matrix and regions 36 of precipitating agent contained on the fibers. The fluid sample in this region contains all of the original lipoprotein components at original lipoprotein concentrations, as indicated.

The central portion of the figure shows a portion 44 of matrix 26, downstream from the reagent reservoir, between the sample-application region 28 and a sample-collection site 32. As the fluid sample is drawn through the web in the reservoir, the deposit on the web is slowly dissolved, releasing precipitating agent into the sample. As the cholesterol-containing particles in the solvent front are drawn toward the sample-collection region to the right in the figure, the aggregated and non-aggregated particles are separated chromatographically. As indicated in the center cutaway portion, LDL and VLDL lipoproteins are largely in precipitated, or aggregated, form.

The right portion of the figure shows a portion of the matrix at the sample-collection site 32. Here the composition of fluid sample which first reaches the sample-collection region is substantially free of non-HDL particles, but contains HDL particles at concentrations present in the originally applied blood sample.

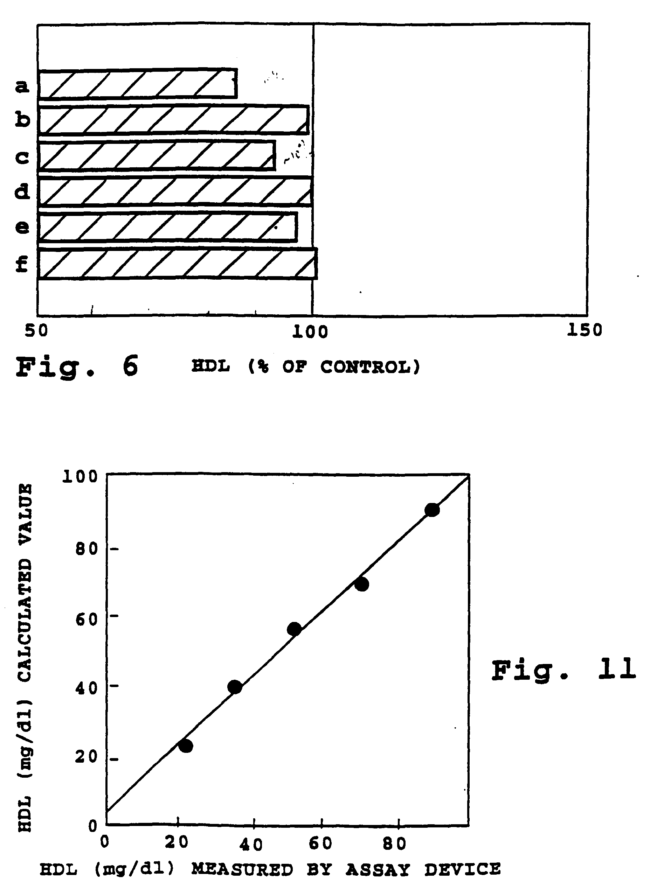

According to an aspect of the invention, it has been discovered that treatment of blood with agents used in selectively precipitating non-HDL blood lipoproteins results in binding of a portion of the HDL present in the sample to glass fibers of the type supplied in commercially available glass filters. The reduction in measured HDL cholesterol resulting from this binding to the glass fibers will be seen below from the data in Figure 6. Such binding can be effectively eliminated by addition of a coating to the fibers of the separation matrix 26.

Two preferred types of coating and their method of preparation are illustrated in Figures 5A-5C.

Figure 5A shows a fragmentary surface portion of a glass fiber 50 having silyl hydroxyl (Si-OH) surface groups, such as groups 52. The silyl hydroxyl groups are typical of glass fiber surfaces in commercial glass filters and the like. Although not shown here, the fiber may also be formulated to contain a small percentage (less than about 2% by weight) of a polymer, such as polyvinyl alcohol (PVA) which is formulated with the glass, prior to formation, to improve the packing properties of the glass fibers.

In the coating method shown in Figure 5B, the glass filter is coated by chemically reacting the surface silyl hydroxyl groups with a hydrophilic silylating agent having the general form $R\text{-}Si(OX)_n$, where R is a hydrophilic group, X is a lower alkyl, preferably methyl or ethyl radical, and n is 2 or 3. Exemplary silylating agents include silyl mono and diethers of bis(hydroxyethyl)aminopropyltriethoxy silane.

The reaction is carried out according to known methods for silylating glass. In a typical procedure, a 2% aqueous "working" solution of the silylating reagent, such as bis(hydroxyethyl)aminopropyl triethoxysilane is prepared from a 62% stock solution obtained commercially, e.g., Huls America (Petrarch Systems, Bristol, PA). The glass filter toning the matrix is dipped into freshly prepared working solution for at least 10 minutes, rinsed with 95% ethanol, and dried in an oven at 50°C for 5 minutes.

As seen in Figure 5B, the silylating reagent can react with either one of two Si-OH surface groups, forming a silyl mono- or di-ether coating, indicated at 54. This coating masks the surface Si-OH groups of untreated glass, replacing these groups with hydrophilic R groups of the type indicated above.

In a second general embodiment, illustrated in Figure 5C, the glass filter is coated with a hydrophilic polymer, such as indicated at 56. In a preferred method for applying the polymer coating, the glass filter forming the matrix is dipped in a solution of polyvinyl alcohol (PVA), followed by drying at elevated temperature (about 90°C) for several minutes. Final incorporation of polymer, as estimated by comparison of mass before and after flame vaporization of the organic compound from the glass filter, is approximately 5% of dry weight. Figure 5C shows the resulting polymer coating 58 formed of polymer fibers. As with the chemical modification method, the coating is effective to mask surface Si-OH groups and replace them with the hydrophilic groups of the polymer subunits.

Preferred polymers for use in the invention include polyvinyl alcohol, polyvinyl pyrrolidine, polyethylene glycol, polypropylene glycol, polyethylenimine, polyvinyl sulfonic acid, poly(3-hydroxybutyric acid), polyacrylic acid, poly(3-hydroxyvaleric acid), poly(4-styrene sulfonic acid), poly(2-acrylamido sulfonic acid, or poly-L-glutamate. Preferred polymer molecular weights are between about 10-200 kilodaltons. The precise preferred polymer content is dependent upon the type of glass fiber matrix, and particularly, its fiber density. As will be seen below, using a Whatman BSB-20 membrane, the preferred polymer content is greater than about 4% polymer, measured as described above, with reference to polyvinyl alcohol as standard.

The effect of various glass compositions and surface coatings on binding of HDL to glass filter fibers can be seen from a study, the results of which are shown in Figure 6. In this study, each of six glass filters, treated as described below, was used as the sieving matrix. In each case, a serum sample previously treated with dextran sulfate precipitating reagent to remove VLDL and LDL, was passed through the filter, then analyzed for HDL-associated cholesterol content by a conventional cholesterol assay. As a control, the same sample was analyzed directly by the solution-phase method, without passage through the filter. MDL cholesterol concentration for the filtered material was then expressed as percent of unfiltered material.

As shown in the top bar (a) in the graph in Figure 6, passage of a serum sample through untreated glass fiber (Whatman BSB-20) resulted in about a 15% loss of HDL cholesterol content, due to binding of HDL to the glass of the matrix. According to manufacturer's specifications, the glass fibers on this filter are formulated with about 1% PVA incorporated into the glass filter during formation, to improve matting properties of the fibers.

The same filter material, after silylation with bis(hydroxyethyl)aminopropyl triethoxysilane, showed almost no binding of HDL, as evidenced by HDL cholesterol content equivalent to that of the control sample (b). The same filter material, when coated with a solution of PVA, at less than 4% polymer by weight (c), exhibited less binding than untreated fibers, but still showed binding of HDL. Filters having a 4-5% PVA coating by weight (d) showed no appreciable HDL binding. From these results it can be determined that for the fiber composition used in this study (Whatman BSB-20), a 4-5% PVA coating was sufficient to prevent binding of HDL to the filter material. Additional studies suggested that the precise amount of PVA coating required is dependent upon the composition and particularly the glass fiber density of the filter material employed.

Also shown in Figure 6 is the result of filtration of sample through glass filters formed by the manufacturer to include 6% PVA by weight. These filters were not specifically surface coated with PVA, according to the methodology described above. Passage of sample through this filter resulted in a slight decrease in HDL-cholesterol content of the filtrate (e). The decreased HDL binding to this filter suggests that the added PVA serves in part to coat and mask Si-OH groups ordinarily on the glass fiber surface. When 6% PVA-augmented fiber material was additionally silylated, using the silylation methodology described above, no appreciable loss of HDL-cholesterol content of sample due to binding of HDL to the filter was observed (f).

The study indicates that both chemical coating by hydrophilic silylation groups and polymer coating by a hydrophilic polymer (at a sufficient polymer concentration) are effective to prevent HDL binding to glass fibers in the presence of precipitating agent.

Completing the description of Figure 1, device 14 includes a test plate 60 composed of an elongate support 62, and multiple wettable, absorbent reaction test pads 64, 66, 68 and 70, carried on the lower surface of the support, at the positions shown. The support is transparent or has transparent windows which allow the test pads to be viewed through the support. The pads in the test plate are attached to the support by a transparent or translucent adhesive material, or by sonic welding or other suitable bonding method. Each pad used in a particular assay contains analyte-dependent reagents effective to produce an analyte-dependent change in the pad which can be detected optically, either visually or by a detector, in a known manner. All or any integral subset of the pads may be employed in a particular assay. The nature of the reagents for cholesterol and triglyceride assays is described below in Sections B and C, below.

Desirably, the reaction test pads are porous, fused polymer or microporous polymer membranes having a thickness, after complete penetration of the fluid, of about 100-150 $\mu$ and side dimensions of about 3 mm. The absorption volume of each pad is preferably between about 0.5-2 $\mu$l. A preferred composition material of the reaction pads is polysulfone.

When the serum reaches the sample-collection sites, such as site 32 adjacent the ends of matrix 26, test plate 60 is moved until pads 64, 66, 68, 70 are in contact with the matrix. In this position, fluid sample in the matrix is drawn into the adjacent pad by capillary flow with fluid movement occurring in a direction normal to the pad surfaces. The plate is held at this position until a desired degree of wetting of the pads is achieved.

The analyte-dependent reagents in the pad produce an analyte-dependent change in the pad which can be detected optically, either visually or by a detector, in a known manner. One preferred apparatus for reading the signal levels of the pads, and for calculating a corrected analyte value based on the readings is described in co-owned U.S. patent application for "Controlled-volume Assay Method and Apparatus", Serial No. 320,474, filed March 8, 1989, incorporated herein by reference.

The test plate 60 is mounted on support 15 by a pair of resilient members, such as elastomeric blocks 71, 72. The blocks act to bias the pads toward a non-transfer position at which the pads are spaced apart from the dispenser's sample-transfer surface, with a spacing typically of between about 0.5 to 1.0 mm.

B. HDL Assay

To operate the device in an assay for determination of HDL in a small blood sample, the sample is placed in well 16 (Figure 1a), and the sample is drawn by capillarity into and through pad 22, yielding a cell-free serum sample which is then drawn into matrix 26 through reservoir 34. As the fluid sample is drawn through the web in the reservoir, the precipitating agent/binder deposit on the reservoir web is slowly dissolved, releasing precipitating agent into the fluid sample. As described above, the reservoir is designed to release the entire amount of precipitating agent over a selected portion of the total solvent flow through the reservoir -- typically over at least the first 10-40% of total solvent flow, and preferably at least the first 20% of flow. The rate of release of the precipitating agent is such as to maintain a concentration of the agent in the fluid sample which is within the range reuqired to selectively precipitate LDL and VLDL present in the sample without precipitating appreciable amounts of HDL.

As the cholesterol-containing particles in the downstream region of the solvent front are drawn into the matrix and toward the sample-collection region to the right in the figure, the aggregated and non-aggregated particles are separated chromatographically, as described above and illustrated in Figure 4. The fluid sample which first reaches the sample-collection region is substantially free of non-HDL particles, but has the same HDL concentration as the cell-free (serum) fluid sample serum which is introduced into the matrix.

Figure 7 is a plan view of the upper surface of matrix 26 in device 14, as viewed through support 62, also showing the four reaction pads carried on test plate 60, and indicated at 64, 66, 68, and 70. The dotted lines 72 in the figure indicate the edges of underlying region 22 and approximate the edges of reservoir 34 in the device, shown in cutaway below matrix 26. The reservoir is designed for slow release of dextran sulfate and $Mg^{2+}$, illustrated as a dried deposit 36 on web fibers 38, as described above. It will be appreciated from the figure that the fluid sample being drawn toward both sides of the matrix is exposed to precipitating agent, so that non-HDL particles on both sides of the matrix are separated from HDL analyte when the fluid sample reaches the sample-collection regions underlying the reaction pads.

Two of the reaction pads in the Figure 7 embodiment are designed for duplicate assay of HDL cholesterol ($HDL_1$ and $HDL_2$), indicated at 66 and 68 in the figure. Two reference standard pads, ($B_1$ and $B_2$, indicated at 64 and 70), provide a self-correcting standard curve for determining cholesterol values, as described in U.S. patent application for "Self-Corrected Assay and Method", Serial No. 396,326, filed August 23, 1989 and incorporated herein by reference. It is appreciated that a number of configurations of the HDL test and reference standard test pads are possible. That is, reference standard pads could alternatively be located at 66 and 68, 64 and 66, 68 and 70, with HDL test pads at the alternate locations in each case. Alternatively, the device can be produced to have only one reaction test pad containing cholesterol-dependent reagents. In addition, the device can be made without a reference standard pad, and standardized reflectance values can be used to determine the concentration of HDL-cholesterol present in the fluid sample tested.

When the serum reaches sample-collection sites (30, 32) adjacent the ends of matrix 26, the test plate is moved toward its sample-transfer position, at which the test pads are in contact with the matrix. At this position, fluid sample in the dispenser is drawn into the pads by capillary flow with fluid movement occurring in a direction normal to the pad surfaces. The plate is held at this position until a desired degree of wetting of the pads is achieved.

The cholesterol-dependent reagents in the test pads produce a change in the pad which can be detected optically, either visually or by a detector, in a known manner. One preferred apparatus for reading the signal levels of the pads, and for calculating a corrected analyte value based on the readings is described in the above-cited co-owned U.S. patent application for "Controlled-Volume Assay Method and Apparatus", Serial No. 320,474, filed March 8, 1989, which has been incorporated herein by reference.

As configured to perform the HDL assay, the above-described apparatus contains both test (HDL) and reference standard pads ($B_1$, $B_2$). The test pads contain common pathway reagent components for converting $H_2O_2$ to a colored signal reaction product. The common pathway components include peroxidase, and a single dye or coupled dye system which is converted by the peroxidase, in the presence of $H_2O_2$, to a distinctively colored signal reaction product. Enzymatic color reactions which employ a variety of substrate-specific oxidases, for enzymatic generation of $H_2O_2$, and subsequent oxidation of a dye to form a colored reaction product are well known. The HDL test pads also contain in addition to the common pathway components, cholesterol esterase for releasing free cholesterol from HDL, and cholesterol oxidase, for producing $H_2O_2$ by reaction with free cholesterol in the sample sample.

The reference standard pads contain, in addition to the common-pathway components, different known amounts of a non-cholesterol reference compound, preferably a D-amino acid, and an oxidase, such as D-amino acid oxidase, for generating $H_2O_2$ when the pad is wet with fluid sample.

The reaction components present in reaction and reference pads used in the HDL assay are summarized in Table 1.

Table 1

| | PAD | |
|---|---|---|
| Components | Reference | HDL |
| HDL Sample (HDL) | + | + |
| cholesterol esterase | | + |
| cholesterol oxidase | | + |
| D-amino acid | + | |
| D-amino acid oxidase | + | |
| peroxidase + dye(s) | + | + |

In the above assay device, the amount of reference compound added to each reference standard is selected to produce, in the presence of a given volume of defined reaction mixture, a signal level corresponding to a known concentration of cholesterol, assayed under identical conditions in the presence of cholesterol oxidase. The actual reference standard produced in the assay will also depend on (a) the amount of soluble interfering compounds present in the serum sample (as distinguished from non-HDL compounds which interfere with the measurement of HDL cholesterol), (b) the condition of the reaction components in the pad, and (c) reaction conditions, including temperature and reaction time.

The signal values of the two reference pads, when plotted as a function of reference compound concentration, gives a two-point standard curve such as the one shown in Figure 8. The signal value is calculated from reflectance or other measured signal properties using known relationships between the measured signal property and plotted signal value. The fact that the curve in Figure 8 does not cross the origin of the graph indicates non-linear inhibition effects.

The signal values measured for the duplicate HDL pads are plotted on the reference standard curve, and the known correspondence between reference standard and cholesterol concentrations is used to determine the cholesterol concentration in each of these pads. It will be appreciated that the cholesterol values so calculated are (a) calculated on the basis of a standard curve, and (b) self-corrected for variations in reaction conditions, and for inhibitory effects and loss of activity of common-pathway components, assuming that all four reaction pads are subject to the same variations in these factors. The concentration of cholesterol associated with HDL can be calculated from the average in cholesterol concentration determined from the two HDL sample pads. Alternatively the cholesterol concentration can be determined from the reflectance value obtained from a single HDL sample pad compared to a single reference standard, or to a standard historically obtained reflectance value.

C. Multi-analyte Lipid Assay

This section describes embodiments of the assay device which are designed for multi-analyte determination of serum components, including HDL cholesterol, and additionally including one or more of the following analytes: total serum cholesterol, LDL cholesterol, and serum triglycerides.

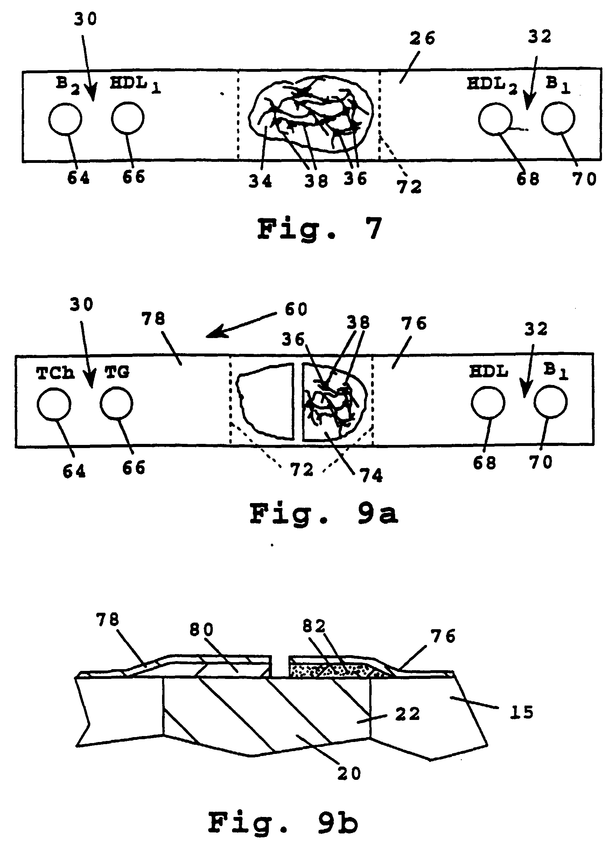

Figure 9a is a plan view of the upper surface of a two-part matrix 76, 78 in the assay device, where the dotted lines 72 again indicate the edges of the sieving pad region underlying the sample application region of the matrix. Fibers 38 and deposits of precipitating agent 36 contained on the fibers are shown in the cutaway of the right side only of matrix 76 which comprises reservoir 74 in this device. The fluid sample which is drawn toward matrix 76 passes through this reservoir, causing a slow release of precipitating agent and precipitation of non-HDL lipoproteins in the sample material. The material flowing from the sieving pad toward the left side of the matrix 78 is not exposed to precipitating agent. Figure 9b shows a fragmentary cross-sectional view of the device in the sample application region, illustrating the division between the two parts of the matrix 76,78, and the presence of precipitating agent 82, indicated by stippling, in the reservoir on the right, but not the left side of the device. The division between the two sides of the reservoir and matrix of the device is illustrated as a space in Figures 9a and 9b; alternatively, a physical barrier, such as a plastic sheet, which is not permeable to the fluid sample fluid and which therefore prevents diffusion between the two parts of the sieving matrix, could be used in the device. The region indicated at 80 on the left side of the device, corresponding to the reagent reservoir may contain a web-like reagent reservoir matrix, or may be occupied by the separating matrix 78. The entire matrix 76, 78 is preferably coated with a hydrophilic polymer or a silylating agent, as described above.

The four test reaction test pads shown in the Figure 9a embodiment are designed for measuring total cholesterol (TCh), triglyceride lipid (TG), and HDL cholesterol (HDL), and include a reference standard test pad ($B_1$). Each

pad contains the above-described common pathway components for converting $H_2O_2$ to a distinctly colored signal reaction product. The reference standard pad contains a known amount of reference compound, such as D-amino acid, and a corresponding oxidase, as described above.

The total cholesterol and HDL test pads each include, in addition to the common pathway components, cholesterol esterase, for releasing esterified cholesterol in free-cholesterol form from serum lipoproteins, including HDL, LDL, and VLDL particles, and cholesterol oxidase, for producing $H_2O_2$ by reaction of with free cholesterol in the fluid sample.

The triglyceride test pad includes, in addition to the common-pathway Components, lipase, L-glycerol kinase, and L-glycerol-3-phosphate oxidase, for generating $H_2O_2$ from triglyceride. These three enzymes function to convert the triglyceride substrate into L-glycerol-3-phosphate, and to oxidize the latter compound, with generation of $H_2O_2$. Since the serum sample drawn into the TG pad contains all of the serum lipoproteins, the TG signal represents total serum triglycerides.

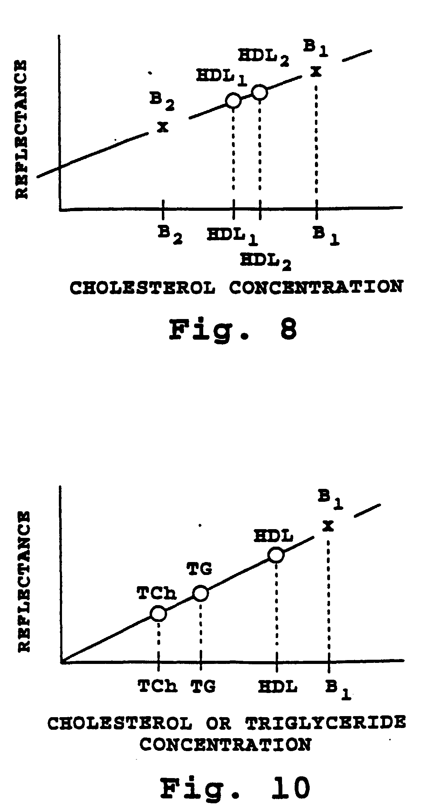

The reference standard pad in the assay is used to construct a single-point standard curve such as shown in Figure 10. The standard curve corresponds to predetermined concentrations of cholesterol and triglyceride, and thus provides a plot for determining analyte concentrations from measured signal values. As described above, the standard curve also functions to correct analyte readings for loss of enzyme activity in the common-pathway components, and for variations in reaction conditions, although non-linear inhibition effects are uncorrected. A two-point standard curve, such as shown in Figure 8, could of course be constructed using a five-pad assay device.

The signal values for total cholesterol, triglyceride, and HDL are plotted on the standard curve to determine self-corrected analyte concentration values for the three analyte pads. From these values, LDL cholesterol can then be calculated from the relationship:

$$\text{LDL cholesterol} = \text{total cholesterol} - \text{total triglycerides}/5 - \text{HDL cholesterol}.$$

From the foregoing it will be appreciated how various objects and features of the invention are met. The assay device provides a simple one-step method for assaying an analyte in a fluid sample containing a selectively precipitable interfering compound, without the need to pretreat or centrifuge the fluid sample. This feature is particularly useful for assaying multiple analytes in small volumes which cannot practically be pretreated to remove interfering compounds. In particular, the method allows a small blood volume, typically less than 50 µl, to be employed in multi-analyte assays in which at least one analyte, e.g., HDL, requires the selective removal of related lipoproteins.

The following example illustrate the preparation and use of assay devices for cholesterol and other lipid testing. The examples are intended to illustrates, but not limit the scope of the invention.

Example 1

Assay for HDL

An assay device like that described in Figure 1 was prepared using the preferred filter materials described in Section A. Stock solution A contained 2 g/100 ml 50,000 dalton dextran sulfate. Stock solution B contained 1M magnesium acetate. To a 1/8" by 5/16" by 150 micron thick glass fiber filter was added 0.75 µl each of stock solutions A and B and 2.21 µl water. The filter was dried for 20 minutes at 50°C and assembled into the device as the precipitating agent-containing reservoir, as described above.

Four test pads in the device contained the reagents described in Section B above. Each reaction pad was prepared from a 150 micron thick polysulfone filter (TR 450; Gelman Sciences, Ann Arbor, MI) cut into 2 x 4 mm rectangles. The two reaction pads for cholesterol determination were infused with 10 µl of a reagent solution containing 150 U/ml cholesterol ester hydrolase, 10 U/ml cholesterol oxidase, 80 U/ml peroxidase, and 20 mM 4-amino-antipyrine (4-AAP) and 80 mM N-ethyl-N-sulfohydroxypropyl-M-toluidine (TOOS), in reduced form, in phosphate buffer at neutral pH. The two reaction pads for the reference standards contained a layer infused with 40 U/ml D-amino acid oxidase, 80 U/ml peroxidase, and 20 mM 4-amino-antipyrine (4-AAP) and 80 mM TOOS, in reduced form, in the phosphate buffer, then dried. Next, 3 µl of 20 mM D-amino acid in ethanol were added, and the pad was dried. The amounts of reference standard were calibrated, in a liquid-phase assay containing a defined reaction buffer, and under defined reaction conditions, and matched with similarly measured cholesterol concentrations from a predetermined volume of known-concentration solution of cholesterol in non-ionic detergent. After drying under reduced pressure, the reaction pads were assembled and attached to the reaction pad plate in the device.

A whole blood sample from a human test subject was obtained conventionally, and applied to the well in the device in an amount sufficient to wet the four test pads. The wetted pads were incubated at room temperature for 90 seconds, or until no further color development was observed.

The analyte-related signal was read by reflectance spectrophotometry, at an illuminating wavelength of 500-700 nm. The values for the reference standard pads were plotted to give a two-point self-corrected standard curve from which the cholesterol readings were calculated.

Example 2

Serum HDL Assay

The assay device used was similar to that described with reference to Figure 1, and was prepared using the reservoir, as described in Example 1, containing a dextran sulfate (MW 50,000)-Mg precipitating agent at neutral pH. The reaction pad formulations were as described in Example 1, for enzymatic determination of cholesterol. The a 1/8" by 5/16" by 150 micron thick glass fiber filter was coated with 5% polyvinyl alcohol by weight, and otherwise used as in Example 1, above.

Five samples were prepared from human serum to contain known amounts of HDL cholesterol, with concentrations varied over the range from 15-100 mg/dL, with evenly spaced dilutions. The HDL cholesterol of all samples were measured in triplicate in the assay device, with results as shown in Table 2:

TABLE 2

| SAMPLE# | MEASURED HDL mg/dL | CALCULATED HDL mg/dL |
|---|---|---|
| 1 | 22.8 | 22.8 |
| 2 | 37.5 | 40.3 |
| 3 | 54.3 | 57.7 |
| 4 | 75.4 | 75.1 |
| 5 | 92.5 | 92.5 |

The calculated or theoretical values (y-axis) were plotted against the results obtained for the samples when measured in the assay device of the invention, as shown in Figure 11.

As shown by this example, the improved assay device of the present invention provides HDL measurements which have the same accuracy as liquid-phase tests, which, in contrast to the present invention, require venous blood samples and involved liquid sample handling.

Example 3

Serum Lipids Assay

The assay device is similar to that described with reference to Figure 9, having a precipitating reagent reservoir in the sample-application region adjacent half the underlying sieving pad only, and is prepared using the reservoir and reaction test pad formulations described in Example 1.

The values for the reaction pads are measured at 500-700 nm as above. The reflectance measurement from the fifth pad is used to construct a standard curve, and this curve is standardized to known cholesterol and triglyceride concentrations as described in Example 1. The signal values from the first four pads are plotted on this curve, and the corrected analyte concentration is read from the corresponding position on the concentration axis.

Total serum cholesterol, HDL, free cholesterol and triglycerides are determined from the standard curve, and these values are used to calculate LDL, as described above.

Although the invention has been described with reference to particular embodiments and configurations, it will be appreciated by one skilled in the art that various changes and modifications may be made without departing from the invention.

**Claims**

1. An assay device for measuring a soluble analyte in a fluid sample which also contains a soluble assay interfering compound which can be precipitated selectively, when exposed to a predetermined concentration of a precipitating agent, said device comprising

a means (16) dimensioned and sized to receive a quantity of said fluid sample,

a sieving matrix in fluid communication with said means (16) dimensioned and sized to receive a quantity of said fluid sample said sieving matrix being capable of separating soluble from precipitated material in such fluid sample, as the sample flows through the matrix from a sample-application site to a sample-collection site in the matrix,

a reagent reservoir which includes the precipitating agent and release means for producing a rate of release of agent from the reservoir into the matrix, as fluid sample flows into and through the matrix, which is effective to produce a concentration of the agent in the fluid sample which is within such predetermined concentration range, and

an assay pad in which fluid sample collected at the sample-collection site can be assayed.

2. An assay device for measuring a soluble analyte in a fluid sample which also contains a soluble assay interfering compound which can be precipitated selectively, when exposed to a predetermined concentration of a precipitating agent, said device comprising

one or more sieving matrices, at least one of said sieving matrices being capable of separating soluble from precipitated material in such fluid sample, as the sample flows through the matrix from a sample-application site to a sample-collection site in the matrix,

a means (16) dimensioned and sized to receive a plurality of said fluid sample,

a reagent reservoir which includes the precipitating agent and release means for producing a rate of release of agent from the reservoir into the matrix, as fluid sample flows into and through the matrix, which is effective to produce a concentration of the agent in the fluid sample which is within such predetermined concentration range, and

an assay pad in which fluid sample collected at the sample-collection site can be assayed.

3. An assay device for measuring a soluble analyte in a fluid sample which also contains a soluble assay interfering compound which can be precipitated selectively, when exposed to a predetermined concentration of a precipitating agent, said device comprising

a sieving matrix capable of separating soluble from precipitated material in such fluid sample, as the sample flows through the matrix from a sample-application site to at least two sample-collection sites in the matrix,

a reagent reservoir which includes the precipitating agent and release means for producing a rate of release of agent from the reservoir into the matrix, as fluid sample flows into and through the matrix, which is effective to produce a concentration of the agent in the fluid sample which is within such predetermined concentration range, and

at least two assay pads in which fluid sample collected at said at least two sample-collection sites can be assayed.

4. An assay device for measuring a soluble analyte in a fluid sample which also contains a soluble assay interfering compound which can be precipitated selectively, when exposed to a predetermined concentration of a precipitating agent, said device comprising

a sieving matrix capable of separating soluble from precipitated material in such fluid sample, as the sample flows through the matrix from a sample-application site to a sample-collection site in the matrix,

a reagent reservoir which includes the precipitating agent and release means for producing a rate of release of agent from the reservoir into the matrix, as fluid sample flows into and through the matrix, which is effective to produce a concentration of the agent in the fluid sample which is within such predetermined concentration range, and

an assay pad in which fluid sample collected at the sample-collection site can be assayed.

5. The device of any of claims 1 to 4, wherein the matrix is composed of a network of fibers, and the reagent reservoir is coated on the fibers.

6. The device of any of claims 1 to 4 wherein the precipitating agent includes a polyanionic compound and a group II metal cation, and the release means is effective to release the polyanionic compound, on contact with fluid sample, at a rate which produces a concentration of the compound effective to selectively precipitate very low density lipoproteins, and low density lipoproteins.

7. The device of claim 6, wherein the polyanionic compound is selected from the group consisting of sulfated polysaccharides, heparin, and phosphotungstic acid, and the class II metal is selected from the group consisting of $Mg^{2+}$, $Mn^{2+}$ and $C^{a+}$.

8. The device of claim 7, wherein the polyanionic compound is dextran sulfate, the class II metal is $Mg^{2+}$, and the concentration of the precipitating agents in the matrix, after contact with the fluid sample, is between about 0.5 and 1.0 mg/ml for dextran sulfate and between about 0.045 to 0.15 M $Mg^{2+}$.

9. The device of claims 1 to 4, wherein the matrix capable of separating soluble from precipitated material in such fluid sample includes a coating material effective to prevent binding of the soluble analyte to the matrix in the presence of such precipitating agent.

10. The device of claims 1 to 4, for use in measuring serum cholesterol associated specifically with high density lipoproteins, wherein the precipitating agent includes a polyanionic compound and a group II metal cation, and the release means is effective to release the polyanionic compound, on contact with fluid sample, at a rate which produces a concentration of the compound effective to selectively precipitate very low density lipoproteins, and low density lipoproteins.

11. The device of claim 10, wherein the polyanionic compound is selected from the group consisting of sulfated polysaccharides, heparin, and phosphotungstic acid, and the class II metal is selected from the group consisting of $Mg^{2+}$, $Mn^{2+}$, and $Ca^{2+}$.

12. The device of claim 9, wherein the polyanionic compound is dextran sulfate, the class II metal is $Mg^{2+}$, and the concentration of the precipitating agents in the matrix, after contact with the fluid sample, is between about 0.5 and 1.0 mg/ml for dextran sulfate and between about 0.045 to 0.15 M $Mg^{2+}$.

13. The device of claim 8, wherein the matrix capable of separating soluble from precipitated material in such fluid sample includes a coating material effective to prevent binding of the high density lipoproteins to the matrix in the presence of such precipitating agent.

14. The device of claim 13, wherein the matrix capable of separating soluble from precipitated material in such fluid sample is composed of a network of glass fibers and the coating material is a hydrophilic polymer.

15. The device of claim 14, wherein said coating is selected from the group consisting of polyvinyl alcohol, polyvinyl pyrrolidine, polyethylene glycol, polypropylene glycol, polyethylenimine, polyvinyl sulfonic acid, poly(3-hydroxybutyric acid), polyacrylic acid, poly (3-hydroxyvaleric acid), poly(4-styrene sulfonic acid), poly(2-acrylamido sulfonic acid), or poly-L-glutamate.

16. The device of claim 10, wherein the matrix is composed of a network of glass fibers having surface silyl groups, and said coating, including the surface silyl groups, is composed of silyl ethers.

17. The device of claim 16, wherein the coating is composed of hydrophilic silanes, especially silyl mono- or di-ethers of bis (hydroxyethyl) aminopropyltriethoxy silane.

18. A method of separating high density lipoproteins from low density lipoproteins and very low density lipoproteins in a blood fluid sample comprising

mixing the sample with a quantity of precipitating agent effective to selectively precipitate the low density lipo-

proteins and very low density lipoproteins, and passing the sample through a matrix of glass fibers, which have a coating which inhibits binding of high density lipoproteins to the fibers.

19. The method of claim 18, wherein the precipitating agent includes a polyanionic compound and a group II metal cation.

20. The method of claim 19, wherein the polyanionic compound is selected from the group consisting of sulfated polysaccharides, heparin, and phosphotungstic acid, and the class II metal is selected from the group consisting of $Mg^{2+}$, $Mn^{2+}$, and $Ca^{2+}$.

21. The method of any of claims 18 to 20, wherein the coating on the matrix is a hydrophilic polymer selected from the group consisting of polyvinyl alcohol, polyvinyl pyrrolidine, polyethylene glycol, polypropylene glycol, polyethylenimine, polyvinyl sulfonic acid, poly(3-hydroxy-butyric acid), polyacrylic acid, poly(3-hydroxyvaleruc acid), poly(4-styrene sulfonic acid), poly(2-acrylamido sulfonic acid), or poly-L-glutamate.

22. The method of any of claims 18 to 20, wherein the matrix comprises glass fibers having surface silanol groups, and said coating, including the surface silanol groups, is composed of silyl ethers.

23. The method of claim 22, wherein the coating is composed of silyl mono- or di-ethers of bis (hydroxyethyl)-aminopropyl-triethoxy silane.

Fig. 1a

Fig. 1b

Fig. 2

Fig. 3a          Fig. 3b          Fig. 3c

Fig. 4

EP 1 028 319 A2

Fig. 5A

R-Si(OX)$_3$ →

Fig. 5B

54

50

Si-O-Si(OX)$_n$-R

Si-O
Si-O
Si(OX)$_n$-R

Si-O-Si(OX)$_n$-R

Si-O
Si-O
Si(OX)$_n$-R

50

Si-OH
Si-OH
52
Si-OH

Si-OH

Si-OH

Si-OH

56 →

Fig. 5C

56

58

Si-OH

Si-OH

Si-OH

Si-OH

Si-OH

Si-OH

Fig. 6    HDL (% OF CONTROL)

HDL (mg/dl) CALCULATED VALUE

HDL (mg/dl) MEASURED BY ASSAY DEVICE

Fig. 11

Fig. 7

Fig. 9a

Fig. 9b

CHOLESTEROL CONCENTRATION

Fig. 8

CHOLESTEROL OR TRIGLYCERIDE
CONCENTRATION

Fig. 10